# EUROPEAN PATENT APPLICATION

(11) **EP 2 781 202 A1**
(43) Date of publication of application: **24.09.2014**
(21) Application number: 14159682.5
(22) Date of filing: 14.03.2014
(51) Int. Cl.: A61C 8/00, A61C 19/04, A61B 5/06

(54) **Implant fixture locator**

(30) Priority: 15.03.2013 US 201361789623 P; 29.10.2013 US 201361896752 P
(71) Applicant: Creative Team Instruments Ltd., 75051 Rishon Le Zion (IL)
(72) Inventor: Taub, Abraham, 75289 Rishon Le Zion (IL)
(74) Representative: ZBM Patents - Zea, Barlocci & Markvardsen

(57) **Abstract**

An apparatus for dental implant fixture location detection that comprises a dental implant fixture location sensor; a disposable protecting cover coupled to the dental implant fixture location sensor; an illumination window configured to illuminate a segment of the gum and adjacent teeth; and a display, showing the depth at which the implant is buried implant.

## Description

The present apparatus generally relates to dental restorative or corrective work. In particular, the present apparatus relates to an apparatus for dental implant fixture location detection for further dental restorative and corrective work.

### BACKGROUND

In the field of dentistry, it is often necessary to replace native teeth by prosthetic teeth, mounted on one or more dental implants to maintain an individual ability to digest food and his or her cosmetic appearance. Dental implants are increasingly used in such procedures. Dental implant is typically composed of a metallic fixture, covered with a metallic plug or cup and it is anchored within the maxillary or mandibular bone. For implant fixture insertion the gum tissue should be opened and then holes are drilled in the patient's jaw and the implant fixtures are fixed, typically screwed, into the holes. The gum tissue is then stretched and sutured over the fixtures. At a later stage the implant fixtures receive a post that bears a thread with the help of which prosthetic teeth are attached to the post and fixed over the post.

Dental practitioners usually insert the post when the implant fixture sufficiently integrates with the recipients' jaw, a process that takes a few months. In order to determine the implant fixture location the practitioner has to make a long cut in gum unveiling the fixtures. The plug is replaced by another plug enabling unobstructed access to the fixtures made. Repeat surgical interventions increase life risk to operation sensitive patients such as patients suffering from diabetes and having poor blood coagulation, or patient suffering from different paroxysmal phenomena such as hypertonia or hypotonia, patients with pacemakers, and others.

Availability of certain procedures and apparatuses enabling precise location of implant fixtures will allow opening a very small area of gum tissue exactly above the surgery cup of each implant fixture. This would help to avoid procedures of cutting and then suturing up of gum tissue, and reduce the number of surgical interventions. WO2011064768 and WO2012147077, to the same assignee and inventor, disclose an apparatus for accurate dental implant fixture location determination including an inductive eddy current effect based dental implant fixture location sensor and a handle.

The existing sensors for detecting hidden under gum surface implant fixtures are generally multi-layer printed circuit boards. Such sensors usually are planar printed circuit boards that have a relatively large outer diameter (about 7 mm), which complicates the search and detection of closely located to each other implant fixtures. Such sensors for example as the one disclosed in WO2012147077 could exhibit certain sensitivity to metal parts (like crowns, implants and metal instruments) located close to the edge of the sensor. In such situations a sensor with smaller diameter could be less sensitive to noise or disturbances that they could cause.

Following detection and determination of the implant fixture location the dentist uses a marker to mark the implant fixture location. Typically, for marking, a marking tool is introduced through a hole in the center of the sensor. The tool applies a mark or a label on the surface of the gum that covers the detected implant fixture location.

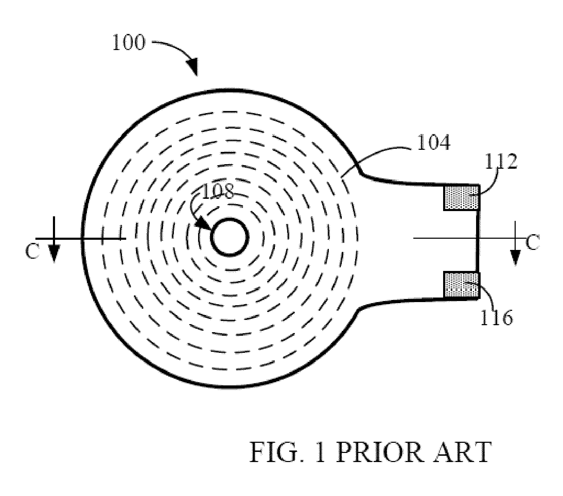

Reference is made to FIG. 1, which is a simplified plan view illustration of an existing sensor for dental implant fixture location determination such as the sensor disclosed in WO2012147077. Dental implant fixture location sensor 100 is a planar Printed Circuit Board (PCB) inductive coil encapsulated into a casing 104 made of a biocompatible material. Sensor 100 includes a centrally positioned opening 108 and a pair of electric contacts 112 and 116. A gum marking blade could be introduced through opening 108 to produce a mark on the gum. Contacts 112 and 116 facilitate electrical communication with control and processing electronics located in handle (not shown), when sensor 100 is inserted into the handle. In some examples dental implant fixture location sensor 100 could also serve as apparatus for implant fixture location determination ON/OFF switch. Insertion of sensor 100 into the handle switches ON the apparatus. Extraction of the sensor from the handle could switch OFF the apparatus, eliminating the need in any dedicated ON/OFF switch.

It is an object of the present disclosure to describe alternative apparatus for dental implant fixture detection enabling precise location of implant fixtures.

### SUMMARY

In a first aspect an apparatus for dental implant fixture location detection is provided. The apparatus comprises a ferrite core dental implant fixture location sensor; and a handle housing at least a display configured to display displacement between a center of dental implant fixture location sensor and a center of dental implant fixture.

In some examples, the apparatus for accurate dental implant fixture location determination may comprise an inductive eddy current effect based dental implant fixture location sensor and a handle. The sensor comprises a ferrite core. The ferrite core may have a central part and a peripheral part. A single-layer coil may be at least partially wound around the central part of the ferrite core. The ferrite core may be configured to concentrate the magnetic field around the windings of the single-layer coil.

In some examples the peripheral part of the ferrite coil could extend at least a fraction of the length of the central part forming a pot shaped ferrite core. In some examples the peripheral part of the ferrite core may not extend along the central part of the ferrite core. In some examples the ferrite core may be a pot shaped ferrite core and in some examples it may be a T-shaped ferrite core.

### BRIEF DESCRIPTION OF THE FIGURES

The sensor and apparatus disclosed are herein presented, by way of non-limiting examples only, with reference to the accompanying drawings, wherein like numerals depict the same elements throughout the text of the specifications. The drawings are not necessarily to scale, emphasis instead being placed upon illustrating the principles of the method and the apparatus.
FIG. 1 is a simplified plan view illustration of an existing sensor for dental implant fixture location determination;
FIGS. 2A and 2B are schematic cross-section views of the dental implant fixture sensor according to some examples;
FIG. 3 is a simplified plan view illustration of an apparatus for dental implant fixture location determination using dental implant fixture sensor of FIG. 2A or 2B; and
FIG. 4 is a simplified plan view illustration of the apparatus for dental implant fixture location determination of FIG. 3 in operation.

### DETAILED DESCRIPTION OF EXAMPLES

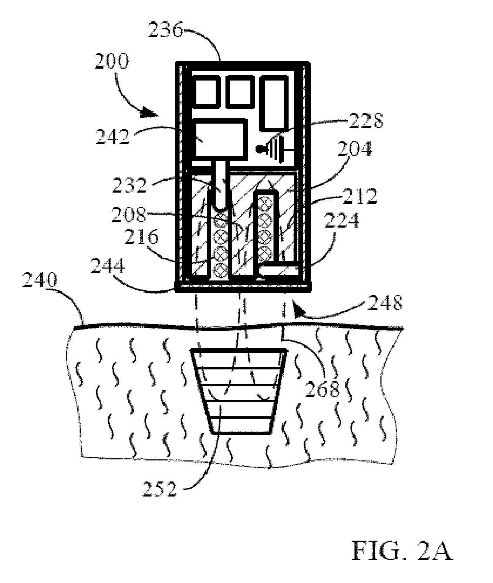

Reference is made to FIG. 2A, which is an example of a dental implant fixture location sensor. Sensor 200 comprises a pot shaped ferrite core 204 with a protruding central part 208 and a peripheral or outer part 212 of the pot shaped ferrite core surrounding the protruding central part 208. Peripheral part 212 of the pot shaped ferrite core 204 is shown as extending on a length similar or equal to the length of protruding central part 208. Although, in some examples, as it will be shown below, the length of outer part 212 of the pot shaped ferrite core 204, could extend on a fraction of the length of protruding central part 208 or may be absent at all thus making ferrite core 204 look like a T-shape. A single-layer coil 216 is wound around a protruding central part 208 of pot shaped ferrite core 204. The pot shaped ferrite core 204 is configured to adjust the ferrite structure to meet the specific requirements related to magnetic field lines 268 and in particular to magnetic field concentration. Pot shaped ferrite core 204 could also include some grooves or channels facilitating coil 216 connections to dental implant sensor electronics 236. Terminal 224 of single-layer coil 216 is connected to a ground 228 common with implant fixture locator electronics. Terminal 232 of coil 216 is connected to the dental implant fixture location sensor electronics 236 and in particular to proximity to frequency converter 242 through another opening or groove in pot shaped ferrite core 204. As mentioned above, pot shaped ferrite core 204 is configured to adjust the ferrite structure to meet the specific requirements of the dental implant fixture location sensing and in particular magnetic field concentration. In addition, pot shaped ferrite core 204 supports high circuit Q-factor.

In order to support better fixture location determination accuracy, the influence of the treatment recipient capacitance on implant fixture location could be neutralized. This could be achieved by connecting the single-layer coil 216 terminals 224 to the ground 228 common with implant locator electronics, where the other single-layer coil 216 terminal 232 is connected, as will be explained below, to implant locator electronics 236. Terminal 224 of single-layer coil 216 could be connected to the common ground 228 through an opening or groove made in pot shaped ferrite core 204. The sensor structure helps sensor 200 or 270 to neutralize the influence of the treatment recipient body capacitance and improves dental implant fixture location detection accuracy. Connected to the ground terminal 224 of single-layer coil 216 is the first winding of single-layer coil 204. The first winding is located between the rest of the windings of sensor coil 216 and the treatment recipient gum 240 and becomes by itself a part of the shield. Terminal 232 is connected to the implant locator electronics 236 and in particular to proximity to frequency converter 242 through another opening or groove in pot shaped ferrite core. Terminal 232 connection to the implant locator electronics closes the chain of a displacement measurement circuit. The displacement measurement circuit could be any of the known and widely used circuits, converting the inductance and the Q-factor of the inductive dental implant fixture sensor to one of the measured parameters like amplitude or frequency, for example such circuit as a known Colpitts Oscillator circuit.

The displacement measurement circuit is configured to generate a frequency signal as a function of proximity or displacement to/from the dental implant fixture. The frequency signal is generated as a function of relative location or displacement between dental implant fixture 252 and dental implant sensor 200. The frequency of the signal grows as the dental implant sensor 200, moved in a scanning movement, approaches the center of the dental implant fixture 252 and reaches its maximum when the dental implant sensor 200 and the dental implant fixture 252 become coaxial. In some examples, the scanning movement of sensor 200 could be in more than one direction.

In one example a thin membrane 244 made of electrically non-conductive and non-magnetic material such as for example, Teflon, polystyrene, epoxy or similar could be used to hermetically close the pot shaped ferrite core opening 248 and prevent moisture and saliva penetration into the single-layer coil and dental implant sensor electronics 236.

The relatively small size of the sensor with outer diameter of less than 4 mm as compared with existing sensors with outside diameter of about 6 to 7 mm reduces sensitivity to other introduced or residing in the mouth metallic objects, improving accuracy of scanned implant fixture location detection.

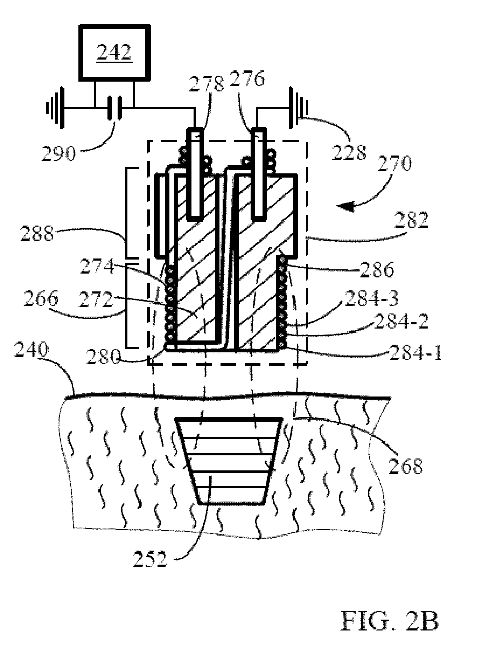

Reference is made to FIG. 2B which is a schematic cross-section of another example of the dental implant fixture location sensor. Sensor 270 includes a T-shaped ferrite core 272 with a segment 266 of a small or first diameter and a segment 288 with a second diameter larger than the small or first diameter and a single-layer coil 274 wound around the segment with small or first diameter. The difference between the first and the second diameter would typically be sufficient to accommodate the windings of the single-layer coil wire. The single-layer coil wire diameter could be about 0.05 to 1.0 mm. The T-shaped ferrite core is configured to concentrate the magnetic field, shown by lines 268 around the windings of the single-layer coil.

Terminal 276 of single coil 274 is connected to a ground 228 common with dental implant sensor electronics 236 (see FIG. 2A), where the other single-layer coil 274 terminal 278 is connected to implant locator electronics 236 and in particular to proximity to frequency converter 242 (see FIG. 2B). Terminal 276 could be connected to the first single-layer coil 274 winding 280, located close to gum 240 through an opening or groove made in ferrite core 272. Winding 280 located close to dental fixture location sensor 270 end facing treatment recipient gum 240 serves as a shield for windings "located remote" from treatment recipient gum 240 and the "located remote" from treatment recipient gum 240 windings are not influenced by treatment recipient capacitance.

Terminal 278 is connected to the implant locator electronics 236 (see FIG. 2A) and in particular to proximity to frequency converter 242 (see FIG. 2A-2B) through another opening or groove in ferrite core 272. Terminal 278 connected to the implant locator electronics 236 closes the chain of a displacement measurement circuit that could be configured to measure the displacement between the implant fixture 252 and the implant fixture sensor 270. Single-layer coils 274 with a reference capacitor 290 form a resonance contour that sets the measurement frequency.

The displacement measurement circuit could be any of the known and widely used displacement measurement circuits, converting the inductance and the Q-factor of the inductive implant fixture sensor 270 to one of the measured parameters like amplitude or frequency. For example, such circuit as a known Colpitts Oscillator circuit could be used for measurements.

Sensor 270 structure supports neutralization of the influence of the treatment recipient or patient capacitance on the implant insert location measurement accuracy, since each next winding 284, 284-1, 284-2 ... 284-n, could serve as a shield between the rest of the windings of single layer sensor coil 274 and the treatment recipient gum 240. Actually, each of the previous windings becomes by itself a part of the shield for the next windings. Winding 286 and other windings "located remote" from treatment recipient gum 240 are not influenced by treatment recipient or patient capacitance.

Sensor 270 could be encapsulated into a casing 282 made of a biocompatible electrically non-conductive and non-magnetic material such as for example, Teflon, polystyrene, epoxy or similar. The encapsulation could hermetically close the ferrite core with all windings and prevent moisture and saliva penetration into the single-layer coil 274 and dental implant sensor electronics 236.

Use of a single-layer coil 274, located on the ferrite core 272 supports construction of the implant fixture sensor 270 with a relatively small outer diameter. The outside diameter of the encapsulated sensor 270 could be less than 4mm. The small size of the encapsulated sensor eliminates the problem of the search and detection of closely located to each other adjacent implant fixtures. The size of the sensor is sufficiently small to support easy marking of the center of implant fixture 252, located in gum 240. It is sufficient to shift a little bit the sensor or to lift it a little bit over the gum 240 to facilitate access to a marking tool. The marking of the center of the dental implant sensor could be done by an existing marking tool.

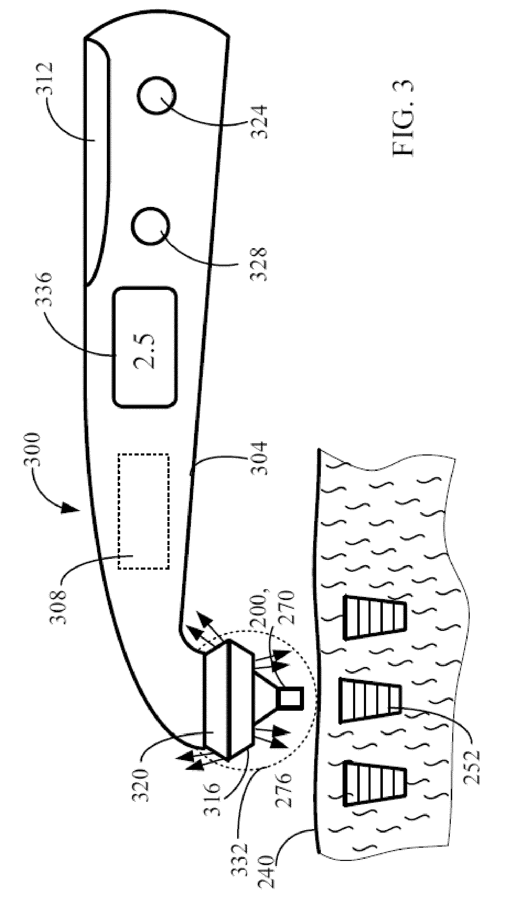

In order to simplify the sterilization and use of sensors 200 and 270 a disposable flexible protective cover 332 could be used to cover the sensors and at least a segment of apparatus 300 (FIG.3) Protective cover could be made of a biocompatible electrically non-conductive and non-magnetic material such as for example, Teflon, polystyrene, polyethylene or similar.

The displacement measurement circuit could be configured to generate a frequency signal as a function of proximity or displacement to/from the dental implant fixture. The frequency signal is generated as a function of proximity or relative location or displacement between the center of dental implant fixture 252 and the center of the dental implant sensor 200 or 270. The frequency of the signal grows as the center of the dental implant sensor 200 or 270 in course of the scanning motion approaches the center of the dental implant fixture 252 and reaches its maximum when the dental implant sensor 200 or 270 and the dental implant fixture 252 become coaxial. Implant fixture locator electronics 236 could be configured to calculate the depth at which the current implant fixture is buried under the gum surface. The implant fixture depth calculations could be based on the maximal detected signal frequency, which is inversely proportional, but not necessary linearly proportional, to the displacement between the center of the dental implant fixture sensor and the center of the dental implant fixture. Practically, it would be the distance between the bottom side of the dental implant fixture sensor and the top/cover of the dental implant fixture. The calculated depth value could be displayed in display 336 in relative or dimensioned units for example, such units as millimeters. The small size of dental implant sensor 200 or 270 facilitates accurate marking of the dental implant fixture center.

The dentist could use the knowledge of the depth or displacement between the dental implant fixture sensor 200 or 270 and the dental implant fixture to improve and shorten the treatment. For example, when the top side of the dental implant fixture is covered by outgrown bone and the dental implant fixture location cannot be identified visually even after making an incision in the gum or tissue covering the dental implant fixture location. The dentist could use the measured depth and order the proper healing cap beforehand as well.

FIG. 3 is a simplified plan view illustration of an apparatus for dental implant fixture location determination using dental implant fixture sensor of FIGS. 2A and 2B. Apparatus 300 includes a convenient to hold handle 304. Residing in the handle 304 electronic circuit 308 could include a control and processing electronics and a memory, and a compartment 312 for a power source. The power source is configured to supply power to all elements of the apparatus 300 and could be a battery. The battery could be a disposable or rechargeable battery. The rechargeable battery could consist of one or more cells and it could be charged through a plug connecting the apparatus to a standard electrical power receptacle.

Apparatus 300 further includes dental implant fixture sensor 200 or 270, an illumination source such as a white LED 316 configured to illuminate the scanned segment of a gum 240 and adjacent teeth, as well as a visual color indicator 320 configured to indicate proximity to the dental implant fixture 252 location, a reset button 324 configured to clear the memory where the previous implant fixture location parameters where recorded, an illumination light ON/OFF switching button 328, and a display 336 configured to display the distance (depth) from the center of the sensor 200, 270 to the center of the dental implant fixture 252. Display 336 could be a one or two digits multi segment LED, bar LED or LCD display. Sensors 200 and 270 could be removable or permanently coupled to handle 304. Visual color indicator 320 configured to indicate proximity (or displacement) to dental implant fixture 252 designed to be easy observed by the dentist.

As explained above, the frequency of the detection signal will reach a maximum when the center of the dental implant fixture sensor will coincide with the center of the dental implant fixture or in other words their axes become coaxial. The highest frequency detection signal associated with the identified implant fixture location could be recorded in the memory of apparatus 300.

The implant fixtures could be buried under the gum at different depth. The highest frequency of the recorded in the memory detection signal is characteristic for a specific implant fixture and if the next implant fixture is buried at a different depth, for example, deeper than the current implant fixture, the next implant fixture will not be found. Apparatus 300 is configured to support renewal of the recorded highest detection frequency signal. Typically, this is performed by operating the reset button 324 to reset the memory, where the highest frequency of the detection signal was recorded or by locating sensor 200 or 270 and operating apparatus 300 proximate to some metallic object. Such object could be a dental scaler or other metal object.

Concurrently with the generation of the detection signal, and in accordance with the displacement of sensor 200 or 270 from the implant fixture, light signal indicator 320 could change its color, for example to a green light to indicate that sensor 200 or 270 of apparatus 300 is being positioned proximate to dental implant fixture 252. Alternatively, additional to green color LEDs could be used to indicate relative displacement between sensor 200 or 270 and implant fixture 252.

The disposable protective cover 332, changed for treatment of the next treatment recipient, could be made of electrically non-conducting, non-metallic material, for example, plastic material. The plastic material could be any bio-compatible plastic material. The disposable protective cover 332 simplifies usage of apparatus 300, although good practice could include some type of cleaning and disinfection of the relevant parts of the apparatus.

A white color LED could be used to illuminate the gum. Illumination of the gum and adjacent teeth is also helpful when scanning dental implant fixtures locations placed deep in the mouth and the visibility is reduced.

Light signal indicator 320 could include one or more Light Emitting Diodes (LED), for example, it could be one or more LEDs emitting light of different colors or one LED configured to emit each of the RGB colors individually and a mix of them. The colors could be selected arbitrarily and be Red, Green, Blue (RGB) or any other color or combination of colors. Significant contrast between the colors could simplify signal identification and decision making. When dental implant fixture sensor 200 or 270 is above an implant location, the color LEDs could begin blinking or change color for example, the LED could lit in Green, when sensor 200 or 270 axis is coaxial with the implant insert axis and change to Blue or other color to indicate that the displacement between the sensor and implant insert axes is growing. An optional audio signal indicator could also be incorporated in the handle. The audio signal indicator could be a simple buzzer configured to become operative when sensor 200 or 270 is above an implant fixture location.

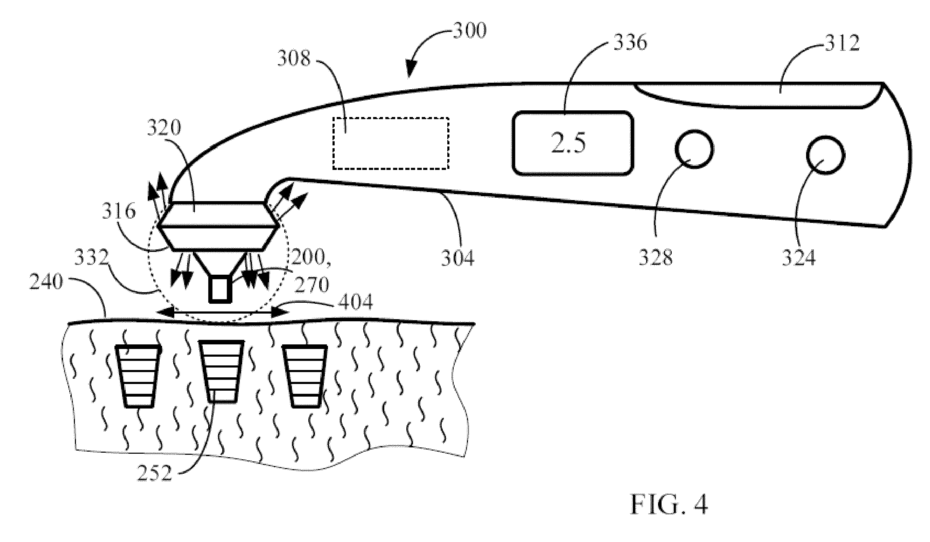

FIG. 4 is a simplified plan view illustration of operation of the apparatus for dental implant fixture location determination of FIG. 3. As shown in FIG. 4, for detection and determination of the dental implant fixture location, apparatus 300 with the dental fixture location sensor 200 or 270 is introduced into a treatment recipient mouth and displaced over gum 240 in a scanning motion as schematically shown by arrow 404. Although, not shown the scanning motion of sensor 200 or 270 could be in more than one direction and the directions could be at different angles to each other. One or more dental implant fixtures 252 have been earlier inserted in the gum. Sensor 200 or 270 is slid over the gum tissue typically being in contact with the gum, which covers the inserted earlier dental implant fixtures location and impedes visual dental implant fixture location detection.

Indicators, such as visual light signal indicators 320 could be employed to signal or indicate the proximity of sensor 200 or 270 (FIG. 2A or 2B) to the target dental implant fixture 252. Light signal indicator 320 and in particular the color of the light emitted by the light signal indicator 320 could be used to indicate the relative proximity of the sensor 200 or 270 with respect to the dental implant fixture 252. For example, a blue color light could be indicative that sensor 200 or 270 is distant relative to dental implant fixture 252 location position, whereas a green light could be indicative that sensor 200 or 270 is being positioned proximate to dental implant fixture 252.

The small diameter of dental implant sensor 200 or 270 facilitates accurate marking of the dental implant fixture center. When based on the light and/or optional audio signal the dentist identifies the dental implant fixture 252 location and marks or labels the dental implant fixture location by shifting a little bit sensor 200 or 270 or lifting it a little bit over the gum 240. The marking could be done by an existing marking tool.

The apparatus described supports fast, reliable, and simple identification of the dental implant fixture location. The dentist has to perform a small incision in order to access a plug covering the fixture and replace it with a similar healing cup or fixture the prosthesis holding abutment.

The dental implant fixture location sensor described is virtually not affected by influence of "uncontrollable factors" such as parasitic treatment recipient body capacitance, temperature inside the treatment recipient mouth, saliva, occasional sensor contacts with tongue or inner side of a cheek, gum nonuniformity, variable and unpredictable capacitance variations caused by scanning movement of the probe occasionally changing the distance between the sensor and the gum, caregiver or dentist capacitance and others. It is also not sensitive to factors that generate electronic "noise" and interfere with the output generated by the sensor.

Although only a number of examples have been disclosed herein, other alternatives, modifications, uses and/or equivalents thereof are possible. Furthermore, all possible combinations of the described examples are also covered. Thus, the scope of the present disclosure should not be limited by particular examples, but should be determined only by a fair reading of the claims that follow.

For reasons of completeness, various aspects of the present invention are set out in the following numbered clauses:
Clause 1. A sensor for dental implant fixture location, said sensor comprising:
   a pot shaped ferrite core with a protruding central part and outer part of the pot shaped ferrite core surrounding the protruding central part;
   a single-layer coil at least partially wound around the protruding central part of the pot shaped ferrite core; and
   wherein the pot shaped ferrite core is configured to concentrate the magnetic field around the windings of the single-layer coil.
Clause 2. The sensor according to clause 1 further comprising an external shield casing surrounding the pot shaped ferrite core with single-layer coil sensor, the external shield casing configured to neutralize influence of a treatment recipient body capacity and/or surrounding metallic parts or instruments on implant fixture location determination accuracy.
Clause 3. The sensor according to clause 1 wherein the a single-layer coil at least partially wound around the protruding central part of the pot shaped ferrite core includes a coreless coil part and wherein the coreless part of the single-layer coil is configured to eliminate influence of the treatment recipient body capacitance by distancing the pot shaped ferrite core edge from scanned gum.
Clause 4. The sensor according to clause 1 wherein a single-layer coil terminal closest to the treatment recipient gum is connected to external shield casing and to a common ground and wherein the other single-layer coil terminal is connected to the measurement circuit.
Clause 5. The sensor according to clause 1 wherein the external shield casing shields components of electronic circuits and sensitive to external influences components.
Clause 6. The sensor according to clause 1 wherein the single-layer coil terminal is connected to implant locator electronics closing the chain of the measurement circuit.
Clause 7. The sensor according to clause 1 wherein a disposable protecting and marking cover configured to leave a marking on the gum, when it is urged against the gum surface with sufficient pressure covers a side of the single-layer coil sensor facing the gum surface covering the implant fixture.
Clause 8. The sensor according to clause 7 wherein the disposable protecting and marking cover configured to leave a marking on the gum is made of non-metallic material.
Clause 9. The sensor according to clause 7 wherein the disposable protecting and marking cover configured to leave a marking on the gum is terminated by a blade type rim.
Clause 10. An apparatus for dental implant fixture location, comprising:
   a dental implant fixture location sensor;
   a disposable protecting and marking cover coupled to the dental implant fixture location sensor and configured to leave a marking on a gum; and
   a white light illumination source configured to illuminate a segment of the gum and adjacent teeth.
Clause 11. The apparatus according to clause 10 wherein the illumination source illuminates the segment of the gum and adjacent teeth by white light is a white LED.
Clause 12. The apparatus according to clause 10 further comprising a display configured to display a distance between the dental implant fixture location sensor and the dental implant fixture.
Clause 13. The apparatus according to clause 10 wherein the sensor is a pot shaped ferrite core with a protruding central part and outer part of the pot shaped ferrite core surrounding the protruding central part and a single-layer coil at least partially wound around the protruding central part of the pot shaped ferrite core and external shield casing shields components of electronic circuits and sensitive to external influences components.
Clause 14. The apparatus according to clause 13 wherein the sensor includes a single-layer coil terminal closest to the treatment recipient gum connected to external shield casing and to a common ground and wherein the other single-layer coil terminal is connected to the measurement circuit.
Clause 15. The apparatus according to clause 13 wherein the single-layer coil terminal is connected to implant locator electronics closing the chain of the measurement circuit.
Clause 16. The apparatus according to clause 10 further comprising visual color indicator configured to indicate proximity to the dental implant fixture location.
Clause 17. The apparatus according to clause 16 wherein the visual color indicator is at least one of a group of indicators consisting of Red LED, Green LED, Blue LED, Orange LED, Yellow LED, and other colors LEDs.
Clause 18. An apparatus for dental implant fixture location, comprising:
   a dental implant fixture location sensor; and
   a display configured to display a distance between the dental implant fixture location sensor and the dental implant fixture.
Clause 19. A sensor for use with a dental implant fixture locator, said sensor comprising:
   a T-shaped ferrite core including a segment of a small diameter (366) and a segment with a diameter larger than the small diameter;
   a single-layer coil wound around the segment with small diameter; and wherein the T-shaped ferrite core and configured to concentrate the magnetic field around the windings of the single-layer coil.
Clause 20. The sensor according to clause 19 wherein terminal of the single-layer coil, connected to the closest to scanned gum wind, is connected to a ground common with dental implant sensor electronics, and the other single-layer coil terminal, connected to the "located remote" from the gum wind, is connected to implant locator electronics.
Clause 21. The sensor according to clause 19 wherein windings located close to the sensor end facing treatment recipient gum serve as a shield for windings "located remote" from treatment recipient gum.
Clause 22. The sensor according to clause 21 wherein the windings "located remote" from treatment recipient gum are not influenced by treatment recipient or patient capacitance.
Clause 23. The sensor according to any one of clauses 19 - 22 wherein the sensor is encapsulated into a casing made of a biocompatible electrically non-conductive and non-magnetic material such as for example, Teflon, polystyrene, epoxy or similar.
Clause 24. The sensor according to any one of clauses 19 - 23 wherein a diameter of the implant fixture sensor is less than 4 mm.
Clause 25. The sensor according to clause 19 wherein the single layer coil and a reference capacitor form a resonance contour.
Clause 26. An apparatus for dental implant fixture location detection, comprising:
   a T-shaped ferrite core dental implant fixture location sensor; and
   a handle housing at least a display configured to display displacement between the center of dental implant fixture location sensor and the center of dental implant fixture.
Clause 27. The apparatus according to clause 26 wherein the displacement measurement circuit cooperates with the T-shaped ferrite core dental implant fixture sensor is configured to generate a frequency signal as a function of displacement from center of dental implant fixture.
Claues 28. The apparatus according to clause 27 wherein the frequency signal is generated as a function of relative location of center of the dental implant fixture and center of dental implant sensor.
Claue 29. The apparatus according to clause 27 wherein the frequency of the signal grows as the center of the dental implant sensor approaches the center of the dental implant fixture and reaches its maximum when the dental implant sensor and the dental implant fixture become coaxial.
Clause 30. The apparatus according to clause 26 wherein the T-shaped ferrite core sensor includes a single-layer coil wound around a segment of the T-shaped ferrite core sensor with small diameter; and wherein one terminal of the single-layer coil is connected to a ground common with dental implant sensor electronics and the other single-layer coil terminal is connected to implant locator electronics measurement circuit.
Clause 31. The apparatus according to clause 26 further comprising a white light illumination source configured to illuminate a segment of a gum and adjacent teeth proximate to the T-shaped ferrite core dental implant sensor and wherein the white light illumination source is a white LED.
Clause 32. The apparatus according to clause 26 further comprising visual color indicators configured to indicate proximity to the dental implant fixture location.
Clause 33. The apparatus according to clause 32 wherein the visual color indicator is at least one of a group of indicators consisting of Red LED, Green LED, Blue LED, Orange LED, Yellow LED, and other colors LEDs.
Clause 34. A disposable protective cover configured to cover at least an implant fixture location sensor and a segment of and apparatus for dental implant insert location determination.
Clause 35. The disposable protective cover according to clause 34 wherein the cover is made from biocompatible electrically non-conductive and non-magnetic material such as Teflon, polystyrene, polyethylene or similar.

## Claims

1. An apparatus for dental implant fixture location detection, comprising:
a ferrite core dental implant fixture location sensor (200; 270); and
a handle (304) housing at least a display (336) configured to display displacement between center of dental implant fixture location sensor (200; 270) and center of dental implant fixture (252).

2. The apparatus according to claim 1, further comprising a displacement measurement circuit configured to generate a frequency signal as a function of displacement from center of dental implant fixture (252).

3. The apparatus according to claim 2 wherein the frequency signal is generated as a function of relative location of center of the dental implant fixture (252) and center of dental implant sensor (200; 270).

4. The apparatus according to claim 2 wherein the frequency signal grows as the center of the dental implant sensor (200; 270) approaches the center of the dental implant fixture (252) and reaches its maximum when the dental implant sensor (200; 270) and the dental implant fixture (252) become coaxial.

5. The apparatus according to any of claims 1-8 wherein the ferrite core is a T-shaped ferrite core (272) comprising a segment with a first diameter (266) and a segment with a second diameter (288) larger than the first diameter; and the dental implant fixture sensor further comprises a single-layer coil (274) wound around the segment with the first diameter; and
wherein the T-shaped ferrite core (272) is configured to concentrate a magnetic field around windings of the single-layer coil (274).

6. The apparatus according to claim 5 wherein a closest to gum terminal (276) of the single-layer coil (274) is connected to a ground (228) common with dental implant sensor electronics (236) and the other single-layer coil (274) terminal (278) is connected to implant locator electronics (236).

7. The apparatus according to claim 5, wherein terminal (276) of the single-layer coil (274), connected closest to gum winding (280), is also connected to a ground (228) common with dental implant sensor electronics (236), and the other single-layer coil (274) terminal (278), connected to located remote from the gum winding (286), is connected to implant locator electronics (236).

8. The apparatus according to claim 5, wherein windings (280) located close to sensor end facing treatment recipient gum (240) serve as a shield for windings (286) located remote from treatment recipient gum (240) and wherein the windings (286) located remote from treatment recipient gum (240) are not influenced by treatment recipient or patient capacitance.

9. The apparatus according to any of claims 1-8 further comprising visual color indicators (320) configured to indicate proximity to the dental implant fixture location.

10. The apparatus according to claim 9, wherein the visual color indicator (320) is at least one LED configured to emit each of RGB colors individually and a mix of them.

11. The apparatus according to any of claims 1-10 wherein the display (336) configured to display displacement between center of dental implant fixture location sensor (200; 270) and center of dental implant fixture (252) is selected from the group of displays consisting of one digit, two digits multi segment LED, bar LED or LCD display.

12. The apparatus according to any one of claims 1 - 11 wherein the implant fixture sensor (270) is encapsulated into a casing (282) made of a biocompatible electrically non-conductive and non-magnetic material such as for example, Teflon, polystyrene, epoxy or similar.

13. The apparatus according to any one of claims 1 - 12 wherein a diameter of the implant fixture sensor (200; 270) is less than 4 mm.

14. The apparatus according to any one of claims 1 - 13 further comprising a reset button (324) configured to reset a memory where a previous implant fixture (252) location parameters were recorded.
